# EUROPEAN PATENT SPECIFICATION

(11) **EP 2 844 215 B1**
(45) Date of publication and mention of the grant of the patent: **11.07.2018**
(21) Application number: 13784732.3
(22) Date of filing: 27.03.2013
(51) Int. Cl.: A61Q 17/00, A61Q 19/10, A61K 8/04, A61K 8/87, A61K 8/898

(54) **FOAMING FORMULATIONS INCLUDING SILICONE-BASED POLYURETHANES**
SCHÄUMENDE FORMULIERUNGEN MIT POLYURETHANEN AUF SILIKONBASIS
FORMULATIONS MOUSSANTES COMPRENANT DES POLYURÉTHANES À BASE DE SILICONE

(30) Priority: 30.04.2012 US 201213460439
(43) Date of publication of application: 11.03.2015
(73) Proprietor: Kimberly-Clark Worldwide, Inc., Neenah, Wisconsin 54956 (US)
(72) Inventor: WOLFF, Kelly Laura, Appleton, Wisconsin 54915 (US); CUNNINGHAM, Corey T., Larsen, Wisconsin 54947 (US); SEIDLING, Jeffery Richard, Appleton, Wisconsin 54915 (US)
(74) Representative: Dehns
(86) International application number: PCT/IB2013/052466
(87) International publication number: WO 2013/164709

(56) References cited:
- EP-A2- 1 078 626
- EP-A2- 2 113 556
- WO-A1-2009/035676
- WO-A2-03/016247
- WO-A2-2008/015344
- DE-A1- 10 223 693
- DE-A1- 10 223 694
- US-A1- 2005 222 001
- US-A1- 2005 265 936
- US-A1- 2007 027 055
- US-A1- 2007 065 383
- US-A1- 2009 214 628
- US-A1- 2010 069 505
- US-B1- 6 482 397
- DATABASE GNPD [Online] MINTEL; March 2008 (2008-03), "Conditioning Mist", XP002747665, Database accession no. 875879
- DATABASE GNPD [Online] MINTEL; September 2011 (2011-09), "Hydrating Serum", XP002747666, Database accession no. 1636066
- DATABASE GNPD [Online] MINTEL; September 2002 (2002-09), "Daily Moisture Therapy Lotion Extension", XP002747667, Database accession no. 10117860

## Description

### BACKGROUND OF THE DISCLOSURE

The present disclosure relates generally to a formulation comprising a silicone-based polyurethane. The silicone-based polyurethane provides the formulation with an improved foaming property. The foaming formulations of the present disclosure are useful as cleansing formulations such as used in alcohol-based hand sanitizers or in hard surface cleansers.

According to the Center for Disease Control, proper cleansing can be one of the most effective steps taken to prevent the spread of diseases and infections. Specifically, proper bodily cleansing according to various sources requires not only using soap but also washing for a sufficiently long period of time in order to remove dirt and any microorganisms that may be present on the skin. For example, the Center for Disease Control has stated that effective cleansing should last at least 15 seconds.

As many consumers fail to effectively cleanse using soaps, alcohol-based sanitizing solutions capable of providing effective antimicrobial sanitation for hand or body cleansing purposes have been developed. Various forms of alcohol-based antimicrobial compositions are known in the art and have been used in the healthcare industry, food service industry, and private sector by individual consumers to provide a convenient means to control and prevent the spread of potentially harmful bacteria and other microorganisms. The alcohol-based antimicrobial compositions are typically utilized to cleanse the skin by destroying bacteria and other microorganisms present thereon, especially on the hands, arms, and face of the user.

In addition to the effective sanitizing, the use of alcohol-based antimicrobial compositions typically does not require subsequent drying of the skin, such as by means of wiping involved in the cleaning process using soap and water. That is, the relatively high alcohol content (e.g., greater than 60% by weight) in the compositions allows for fast and essentially complete evaporation of the liquid components of the composition from the skin.

Conventionally, these alcohol-based antimicrobial compositions further include viscosity increasing agents to prevent runoff and to increase residence time on the skin. Such products are known as alcohol gels. While longer residence time tends to enhance antimicrobial action, it also tends to magnify certain undesirable side effects. In particular, frequent use of alcohol gels may cause skin irritation and dryness. This can be a problem for health care professionals, child care providers, food service workers and others who use alcohol gels to disinfect or sanitize their hands multiple times in a day.

To counteract the drying and irritating effects of alcohol gels, foaming alcohol formulations including conventional ethoxylated silicones or conventional fluorinated compounds have been used. However, these agents used to create the foam in these formulations may cause the formulations to have poor aesthetics and/or skin feel properties, as well as poor foam stability. In addition, conventional fluorinated compounds impose potential environmental and safety concerns.

Accordingly, there is a need for a foaming formulation that provides effective skin cleansing and sanitizing effects, while having good foam stability. It would further be advantageous if the foaming formulations provided improved aesthetic properties and foaming appearance, while maintaining high antimicrobial capacity.

### BRIEF DESCRIPTION OF THE DISCLOSURE

It has now been unexpectedly found that cleansing foaming formulations can be formed without the use of conventional ethoxylated silicones and conventional fluorinated compounds. Particularly, the foaming formulations of the present disclosure include silicone-based polyurethanes that are surprisingly highly efficient foaming agents for hydro-alcoholic solutions. Specifically, the silicone-based polyurethanes surprisingly provide outstanding foam when used in hydro-alcoholic solutions having high alcohol content (e.g., greater than 50%, and typically above 70%, by weight of alcohol).

The foam of a foaming formulation is a two phase system comprised of gas cells, or bubbles, that are surrounded by a thin, continuous liquid film phase. The amount of liquid in the thin film defines the type of foam that is generated. For example, conventional detergent solutions and foaming alcohol sanitizer formulations are classified as "wet foams". In a wet foam, the liquid is mainly found in the junctions between gas bubbles, known as "Plateau borders". As the amount of liquid increases relative to the amount of gas bubbles, the thin liquid films surrounding the bubbles and the Plateau borders swell, forcing the bubbles to take a spherical shape. As the swelling increases, the borders lose rigidity and fall apart, causing the foam to degrade into a bubbly liquid that has no overall order or rigidity.

In completely aqueous foams, conventional foaming agents (e.g., conventional ethoxylated silicones and fluorinated compounds) are concentrated at the surfaces of the gas bubbles. This organization stabilizes the thin liquid films against rupture. In hydro-alcoholic foaming formulations, such as foaming alcohol sanitizing formulations, however, the presence of the alcohol affects the organization of the conventional foaming agents and the rate of evaporation of the formulation becomes a significant variable. It is believed that these two factors neutralize the ability of conventional foaming agents to create and stabilize foam.

It has now been unexpectedly discovered that specific amphiphilic terpolymers can overcome the stability problems to generate a high quality, stable foam in hydro-alcoholic solutions. Further, it has been discovered that the polymeric nature of the foaming formulations of the present disclosure provide enhanced skin aesthetics (e.g., substantivity while formulation is in use, reduced tackiness during and after formulation use, soft and conditioned skin feel of formulation, and improved foam appearance).

Accordingly, the present disclosure is directed to a foaming formulation comprising water, an alcohol, and a silicone-based polyurethane having a polymeric backbone comprising at least one silaphilic moiety, at least one lipophilic moiety and at least one hydrophilic moiety; wherein the alcohol is present in the formulation in the amount of at least 50% by weight; wherein the polymeric backbone comprises a bis-(PEG)x dimethicone, wherein x ranges from 8 to 20, and the at least one lipophilic moiety includes a diisocyanate monomer.

The present disclosure is further directed to a process of preparing a foaming formulation. The process comprises mixing water, an alcohol, and a silicone-based polyurethane having a polymeric backbone comprising at least one silaphilic moiety, at least one lipophilic moiety and at least one hydrophilic moiety; wherein the alcohol is present in the formulation in the amount of at least 50% by weight; wherein the polymeric backbone comprises a bis-(PEG)x dimethicone, wherein x ranges from 8 to 20, and the at least one lipophilic moiety includes a diisocyanate monomer.

The present disclosure is further directed to a method for cleansing including applying to a substrate a foaming formulation. The formulation comprises water, an alcohol, and a silicone-based polyurethane having a polymeric backbone comprising at least one silaphilic moiety, at least one lipophilic moiety and at least one hydrophilic moiety; wherein the alcohol is present in the formulation in the amount of at least 50% by weight; wherein the polymeric backbone comprises a bis-(PEG)x dimethicone, wherein x ranges from 8 to 20, and the at least one lipophilic moiety includes a diisocyanate monomer.

Other objects and features will be in part apparent and in part pointed out hereinafter.

### DETAILED DESCRIPTION OF THE DISCLOSURE

The present disclosure is directed to a foaming formulation comprising water, an alcohol, and a silicone-based polyurethane. The foaming formulation provides an improved foaming effect providing effective cleansing and sanitization, while further providing improved aesthetics and, in some embodiments, skin-feel. Surprisingly, the formulation has improved foam stability as compared to foaming formulations including conventional ethoxylated silicones and conventional fluorinated compounds.

Typically, the foaming formulations of the present disclosure include from 5% by weight to 45% by weight water, including from 10% by weight to 40% by weight, and including from 20% by weight to 30% by weight.

Additionally, the foaming formulations include an alcohol. Any C₁-C₆ alcohol typically used in the formulation art for providing antimicrobial activity, thereby killing and/or inhibiting the growth of bacteria and other microorganisms, can suitably be used in the formulations of the present disclosure. Particularly suitable alcohols include methanol, ethanol, n-propanol, isopropanol, n-butanol, tert-butanol, 2-butanol, pentanol, hexanol, or combinations thereof.

The alcohol is present in the foaming formulations in an amount of at least 50% by weight, such as from 50% by weight to 90% by weight, including from 60% by weight to 80% by weight, and including from 65% by weight to 75% by weight.

In addition to the water and alcohol, the hydro-alcoholic foaming formulations of the present disclosure include a silicone-based polyurethane. The silicone-based polyurethane used in the formulations includes a polymeric backbone having at least one silaphilic moiety, at least one lipophilic moiety and at least one hydrophilic moiety. As used herein, "silaphilic moiety" refers to a silicone-soluble group (e.g., dimethyl siloxane). A further description of silaphilic moieties can be found in Anthony J. O'Lenick, Silicones For Personal Care, Allured Publishing Corporation, Carol Stream, IL (2003), Ch. 16 & 17 (ISBN 0-931710-97-9). As used herein, "lipophilic moiety" refers to an oil-soluble group (e.g., cyclohexyl). As used herein, "hydrophilic moiety" refers to a water-soluble group (e.g., ethylene oxide). These different moieties have different solubilities in the hydro-alcoholic solution, allowing for the enhanced stabilization of foam in the formulation. Also, the polymeric nature of the silicone-based polyurethane provides enhanced skin aesthetics and skin feel.

The hydrophilic moiety includes the compound bis-(polyethylene glycol)ₓ dimethicone, also referred to as Bis-PEG-X Dimethicone, wherein x ranges from 8 to 20, including a range of from 10 to 17, and including from 12 to 16. In one embodiment, the hydrophilic moiety is Bis-PEG-15 Dimethicone.

The lipophilic moiety used in the backbone of the silicone-based polyurethane includes any diisocyanate monomer, and particularly, isophorone diisocyanate (IPDI). Another suitable lipophilic moiety for use includes hexamethylene diisocyanate (HDI). Still other suitable lipophilic moieties for use in the backbone of the silicone-based polyurethane include saturated methylene diphenyldiisocyanate (i.e., dicyclohexylmethane diisocyanate) (SMDI), methylene diphenyl diisocyanate (MDI) and toluene diisocyanate (TDI).

One particularly suitable silicone-based polyurethane is prepared by reacting diisocyanate monomers, such as isophorone diisocyanate, with Bis-PEG-X Dimethicone, wherein x ranges from 8 to 20. One exemplary silicone-based polyurethane of this embodiment is Bis-PEG-15 Dimethicone/IPDI copolymer (CAS # 190793-18-1), commercially available as Polyderm PPI-SI-WS, from Alzo International Corporation (Sayreville, New Jersey).

Another suitable silicone-based polyurethane is prepared to include Bis-PEG-X Dimethicone, wherein x ranges from 8 to 20, and hexamethylene diisocyanate. One exemplary silicone-based polyurethane of this embodiment is Bis-Methoxy PEG-10 Dimethyl MEA/HDI/Bis-PEG-10 Dimethicone copolymer, commercially available as Microcare Silicone C1024, from Thor Personal Care, France).

The foaming formulations may include the silicone-based polyurethanes in amounts of from 0.1% by weight to 10% by weight, including from 0.5% by weight to 5.0% by weight, and including from 1.0% by weight to 3.5% by weight. In particularly suitable embodiments, the foaming formulations include from 0.5% to 3.0% by weight silicone-based polyurethane.

In addition to the above, the foaming formulation may also include various optional agents to modify the physical, chemical, hedonic or processing characteristics of the formulations or serve as beneficial agents when used for a targeted purpose or in a targeted user population. The optional agents include, for example, emollients, humectants, moisturizers, botanicals, foam stabilizers, vitamins, disinfectants, abrasives, non-aqueous solvents, preservatives, pH modifiers, sequestrants, antimicrobials, antioxidants, anti-reddening agents, astringents, deodorants, external analgesics, film formers, fragrances, hydrotropes, opacifiers, skin conditioning agents, skin exfoliating agents, skin protectants, sunscreens, thickeners.

Generally, emollients lubricate, soothe, and soften the skin surface. Exemplary emollients include oily or waxy ingredients such as esters, ethers, fatty alcohols, hydrocarbons, silicones, and combinations thereof. Particular emollients that may be used include ethoxylated and propoxylated alcohols, such as cetyl alcohols and ethoxylated lanolin.

Particular moisturizers could include, but are not to be limited to, PEG-7 glyceryl cocoate, isopropyl palmitate, C₁₂-C₁₅ alkyl benzoate, natural oils such as jojoba oil, synthetic oils such as mineral oil, silicones such as dimethicone, fatty alcohols and acids such as cetyl alcohol and stearic acid, waxes such as beeswax. One skilled in the art will recognize that this list is not all inclusive and could include any other suitable materials commonly known in the art or referenced in the Personal Care Products Council (PCPC) Compilation of Ingredients Used in Cosmetics in the United States (CIUCUS).

Humectants are hydroscopic agents that are widely used as moisturizers. Their function is to prevent the loss of moisture from the skin and to attract moisture from the environment. Common humectants include, for example, glycerin, propylene glycol, butylene glycol, betaine, sodium hyaluronate, sorbitol, urea, hydroxyethyl urea, and combinations thereof.

Exemplary disinfectants for use in the foaming formulations include triclosan, quaternary ammonium compounds, phenolics, and essential oils having antimicrobial action (e.g., thyme, eucalyptus, neem), and combinations thereof.

Another additive for use in the foaming formulation may be one or more non-aqueous solvents. Although not required, non-aqueous solvents may aid in dissolving certain components (e.g., preservatives, anti-microbial agent). Examples of some suitable non-aqueous solvents include, but are not limited to, glycerin, glycols, such as propylene glycol, butylene glycol, triethylene glycol, hexylene glycol, polyethylene glycols, ethoxydiglycol, and dipropyleneglycol, triglycerides, ethyl acetate, acetone, triacetin, and combinations thereof.

Preservatives for increasing the shelf life of foaming formulations may also be used. Exemplary suitable preservatives include, but are not limited to, Kathon CG, which is a mixture of methylchloroisothiazolinone and methylisothiazolinone available from Dow Chemical Company, Midland, Michigan; Mackstat H 66 available from Rhodia, member of the Solvay Group, Bristol, Pennsylvania; DMDM hydantoin (e.g., Glydant Plus, Lonza, Inc., Switzerland); tetrasodium EDTA; iodopropynyl butylcarbamate; benzoic esters (parabens), such as methylparaben, propylparaben, butylparaben, ethylparaben, isopropylparaben, isobutylparaben, benzylparaben, sodium methylparaben, and sodium propylparaben; 2-bromo-2-nitropropane-1,3-diol; benzoic acid; amidazolidinyl urea; diazolidinyl urea. Other suitable preservatives include those sold by Ashland Inc., Ashland, Kentucky, such as "Germall 115" (amidazolidinyl urea), "Germall II" (diazolidinyl urea), and "Germall Plus" (diazolidinyl urea and iodopropynyl butylcarbonate).

Suitable skin conditioning agents for use in the foaming formulations include quaternium and polyquaternium ingredients. Particularly suitable quaternium ingredients include behentrimonium chloride, behentrimonium methosulfate, cetrimonium chloride, cocotrimonium chloride, quaternium-79 hydrolyzed collagen, quaternium-80, quaternium-88, quaternium-95, and C₁₀-C₄₀ isoalkylamidopropylethyldimonium ethosulfate. Particularly suitable polyquaternium ingredients include polyquaternium-2, polyquaternium-7, polyquaternium-10, polyquaternium-68, polyquaterium-78, polyquaternium-82, and polymethylacrylamidopropyltrimonium chloride.

In general, the pH of the foaming formulations may be controlled to be within any desired range, depending on the targeted use. For bodily cleansing, it is typically desirable to have a foaming formulation with a neutral pH. If necessary, various pH modifiers may be utilized in the foaming formulation to achieve the desired pH level. For instance, some examples of basic pH modifiers that may be used in the formulations of the present disclosure include, but are not limited to, ammonia; mono-, di-, and tri-alkyl amines; mono-, di-, and tri- alkanolamines; alkali metal and alkaline earth metal hydroxides; alkali metal and alkaline earth metal silicates; and mixtures thereof. Specific examples of basic pH modifiers are ammonia; sodium, potassium, and lithium hydroxide; sodium, potassium, and lithium meta silicates; monoethanolamine; triethylamine; isopropanolamine; diethanolamine; and triethanolamine.

Moreover, some examples of acidic pH modifiers that may be used in the present disclosure include, but are not limited to, mineral acids; and carboxylic acids; and polymeric acids. Specific examples of suitable mineral acids are hydrochloric acid, nitric acid, phosphoric acid, and sulfuric acid. Specific examples of suitable carboxylic acids are citric acid, glycolic acid, lactic acid, maleic acid, malic acid, succinic acid, glutaric acid, benzoic acid, malonic acid, salicylic acid, gluconic acid, and mixtures thereof. Specific examples of suitable polymeric acids include carrageenic acid, humic acid, fulvic acid, and alginic acid.

In one embodiment, the foaming formulation may additionally include one or more sequestrants. A sequestrant is a substance whose molecules can form one or more bonds with a metal ion. In particular, water often contains metal ions, such as calcium ions, that might react with anionic components (e.g., acids) present within the foaming formulation. For example, in one embodiment, an anionic component that remains substantially unreacted with metal ions can better function as a cleansing agent. Some examples of sequestrants that may be used in the foaming formulations of the present disclosure include, but are not limited to, ethylenediamines, ethylenediaminetetraacetic acids (EDTA) acid and/or salts thereof, citric acids and/or salts thereof, glucuronic acids and/or salts thereof, iminodisuccinic acid and/or salts thereof, polyphosphates, organophosphates, dimercaprols.

In order to better enhance the antimicrobial efficacy of the foaming formulation, other optional antimicrobial agents (e.g., chlorohexidine digluconate, polyhexamethylene biguanide (PHMB), benzalkonium chloride, benzethonium chloride, cetyl pyridinium chloride) can be included in the formulation.

Other optional agents include: antioxidants (product integrity); anti-reddening agents, such as aloe extract; astringents--cosmetic (induce a tightening or tingling sensation on skin); astringents--drug (a drug product which checks oozing, discharge, or bleeding when applied to skin or mucous membrane and works by coagulating protein); botanicals (e.g., Actiphyte of Aloe Vera 10 Fold GL, Actiphyte of Cucumber GL, Actiphyte of Japanese Green Tea GL, all from The Lubrizol Corporation, Wickliffe, Ohio); foam stabilizers (e.g. Polyox WSR 205 from Dow Chemical Company, Midland, Michigan); vitamins (e.g., tocopheryl acetate, retinyl palmitate, panthenol); deodorants (reduce or eliminate unpleasant odor and protect against the formation of malodor on body surfaces); external analgesics (a topically applied drug that has a topical analgesic, anesthetic, or antipruritic effect by depressing cutaneous sensory receptors, of that has a topical counterirritant effect by stimulating cutaneous sensory receptors); film formers (to hold active ingredients on the skin by producing a continuous film on skin upon drying); fragrances (consumer appeal); hydrotropes (helps dissolve some anti-microbial agents); opacifiers (reduce the clarity or transparent appearance of the product); skin conditioning agents; skin exfoliating agents (ingredients that increase the rate of skin cell turnover such as alpha hydroxy acids and beta hydroxyacids); skin protectants (a drug product which protects injured or exposed skin or mucous membrane surface from harmful or annoying stimuli); sunscreens and thickeners (to increase the viscosity of the formulation).

The amounts of the optional components will depend on the formulation to be prepared and the amounts of the other components in the foaming formulation.

In one particularly suitable embodiment, the foaming formulations of the present disclosure are substantially free of agents that are capable of forming micelles. In this context, and unless otherwise specified, the term "substantially free" means that the foaming formulations contain less than a functional amount of micelle-forming agents, typically less than 1.0%, including less than 0.5%, including less than 0.1%, and also including zero percent, by weight of micelle-forming agents.

Micelle-forming agents, non-limiting examples of which include conventional ethoxylated silicones (e.g., Bis-PEG(10-20) dimethicones), 3-(3-hydroxypropyl)heptamethyl-trisiloxane, polysiloxane betaine, and polyalkylene-modified siloxane block copolymers, used to create foam in conventional foaming formulations can cause the formulations to have poor aesthetics and/or skin feel properties such as a sticky, soapy, and/or tacky skin feel. Further, these conventional foaming formulations have poor foam stability (e.g., bubbly liquid having no overall order, no rigidity, etc.). Accordingly, by avoiding the use of these agents in the foaming formulation of the present disclosure, the foaming formulation provides improved cleansing and/or sanitizing to the skin of the user, and further has improved foam stability.

### METHODS OF PREPARING THE FOAMING FORMULATIONS

The foaming formulations are generally prepared by mixing all components together to form a homogeneous solution. Typically, the foaming formulation is prepared by mixing the water, alcohol, and silicone-based polyurethane (and, additionally, any other optional agents) together minimizing aeration. In one embodiment, the water, alcohol, and silicone-based polyurethane are mixed at room temperature.

Suitably, any solid components are first completely dissolved in water or other solvents before mixing with other components. The foaming formulation can be dispensed from a pump or aerosol as generally available in the art.

### METHODS OF USE

The foaming formulations of the present disclosure can be used to provide effective cleansing and/or sanitizing of animate and inanimate surfaces. In one embodiment, the foaming formulations may be used to clean and/or sanitize a user's body. Particularly, in one suitable embodiment, the foaming formulations are in the form of liquid or gel formulations capable of being topically applied to the skin of a user to kill and/or inhibit the growth of bacteria and other microorganisms on the skin, particularly, on the hands, arms, and face of a user.

Moreover, the foaming formulations have improved stability such that the foaming formulations provide a cleansing effect for a sufficiently long period of time in order to effectively remove dirt and microorganisms. The foaming formulations have improved aesthetics (e.g., soft feel, improved foam appearance (e.g., even dispersion of foam from a dispensing device, smaller, more densely packed gas bubbles having uniform size and density, gas bubbles that do not degrade or convert quickly to a bubbly liquid)) and skin-feel.

Having described the disclosure in detail, it will be apparent that modifications and variations are possible without departing from the scope of the disclosure defined in the appended claims.

### Examples

The following non-limiting Examples are provided to further illustrate the present disclosure.

### Example 1

In this Example, a suitable foaming formulation of the present disclosure was prepared. The components of the formulation are shown in the table below.

| **Component** | **INCI Name** | **Wt%** |
|---|---|---|
| Water | | 25.25 |
| Polyderm PPI-SI-WS | Dimethicone Copolyol/IPDI Copolymer | 1.50 |
| Polyox WSR 205 | PEG 14M | 0.10 |
| (Dow Chemical Company, Midland, Michigan) | | |
| Ethyl Alcohol (Grain Processing Corporation, Muscatine, Iowa) | Ethyl Alcohol | 67.20 |
| Isopropyl Alcohol (Ashland, Inc., Ashland, Kentucky) | Isopropyl Alcohol | 5.05 |
| Betaine | Betaine | 0.50 |
| (Ashland, Inc., Ashland, Kentucky) | | |
| Actiphyte of Aloe Vera 10 Fold GL | *Aloe Barbadensis* Leaf Extract; Water; Glycerin | 0.10 |
| (The Lubrizol Corporation, Wickliffe, Ohio) | | |
| Actiphyte of Cucumber GL | *Cucumis Sativus* (Cucumber) Fruit Extract; Water; Glycerin | 0.10 |
| (The Lubrizol Corporation, Wickliffe, Ohio) | | |
| Actiphyte of Japanese Green Tea GL | *Camellia Oleifera* Leaf Extract; Water; Glycerin | 0.10 |
| (The Lubrizol Corporation, Wickliffe, Ohio) | | |
| Vitamin B5 | Panthenol | 0.10 |
| (DSM, The Netherlands) | | |
| Purac FCC88 (Purac, Lincolnshire, Illinois) | Lactic Acid | q.s. |
| **Total** | | **100.00** |

To prepare the formulation, water was first added to a vessel. While stirring the water, Polyox WSR 205 was slowly added and mixed until it was completely dissolved. The silicone-based polyurethane, plant extracts, betaine, panthenol, and lactic acid were then added to the solution. Finally, ethanol and isopropyl alcohols were added, and the mixture was mixed to homogeneity at room temperature. The resulting formulation was clear in appearance.

### Example 2

In this Example, formulations including a silicone-based polyurethane as the foaming agent were evaluated for aesthetics and foam appearance.

Two formulations (Codes A and B) were prepared by mixing a silicone-based polyurethane, Dimethicone Copolyol/IPDI Copolymer (available as Polyderm PPI-SI-WS from Alzo International Corporation (Sayreville, New Jersey)), with PEG 14M and betaine as shown in the table below. Further, a comparative foaming formulation including a Silsurf Di-1010 (Bis-PEG-10 Dimethicone) (available from Siltech Corporation, Ontario, Canada) (Code M) was prepared.

| Code | A | B | M |
|---|---|---|---|
| Polyderm PPI-SI-WS | 1.50 | 1.50 | -- |
| Polyox WSR 205 (Dow Chemical Company, Midland, Michigan) | 0.10 | 0.10 | -- |
| Betaine | 0.50 | 0.25 | 0.50 |
| Silsurf di-1010 (Bis-PEG-10 Dimethicone) (Siltech Corp., Ontario, Canada) | -- | -- | 2.6 |

Eight panelists evaluated the two test formulations (Codes A & B) for aesthetics/feel and foam appearance. Specifically, the panelists were to use the foaming formulation and rate them, based on aesthetics/feel and foam appearance (e.g., substantivity while in use, reduced stickiness/tackiness during and after use, soft and conditioned skin feel after formulation has dried, uniform size of foaming bubbles, compactness of foam, even flow rate of foam), from most preferred to least preferred. Then, the panelists were to compare their most preferred formulation with the comparative foaming formulation of Code M. Again, the panelists were to use both their preferred silicone-based polyurethane-containing foaming formulation and the Code M comparative formulation and compare the formulations based on aesthetics/feel and foam appearance.

Prior to use of the foaming formulations, and in between uses of various formulations, the panelists were to thoroughly rub and rinse hands with warm water for 15 seconds. The formulation to be used was first consistently and slowly pumped from its dispenser for 5 pumps of foam into the sink prior to evaluation to ensure the mesh pump was warmed up. Then, to evaluate the formulation, 1-2 pumps of foam were slowly and consistently pumped into the panelist's hands. The panelists observed foam appearance in their hands. Further, as the formulation was spread around their hands, the panelists evaluated the aesthetics and feel of the formulation during use.

The results of the comparison between the silicone-based polyurethane-containing formulations and the comparative Code M formulation are shown in the table below.

| Code A v. Code M | | Code B v. Code M | |
|---|---|---|---|
| A | M | B | M |
| A | M | B | M |
| A | M | | |

| | | | |
|---|---|---|---|
| *The code that received the greater acceptance is bolded and underlined. | | | |

As shown, the silicone-based polyurethane-containing formulations under Code A and Code B were both preferred over the Code M formulation. Particularly, Code A was preferred over Code M 66% of the time, and Code B was preferred over Code M 100% of the time. Accordingly, this Example shows that the silicone-based polyurethane is more preferred as the foaming agent in a foaming formulation.

### Example 3

In this Example, the foaming formulation of Example 1 was prepared and evaluated for its disinfecting ability against *E. coli.*

Specifically, the foaming formulation was evaluated using disinfectant suspension testing based on EN1500 (1997), available at http://wwww.cen.eu/cen/Products/EN/Pages/default.aspx. Initially, each of the left and right hands of 8 test subjects were swabbed and analyzed for the presence of *E. coli* (prevalue).

Each of the left and right hands of the 8 test subjects were then treated with 8 pumps of the foaming formulation. The formulation was then rubbed into the respective hand for a period of about 30 seconds. A second treatment of 8 pumps of the foaming formulation was then again applied to the left and right hands and the formulation was rubbed into the hands for a period of about 30 seconds.

The hands were then swabbed and analyzed for presence of *E. coli* (postvalue). The swabbed samples were diluted according to the method of EN1500. The disinfectant ability of the test foaming formulation was compared to the disinfectant ability of the EN1500 Reference formulation (70% isopropyl alcohol in water). The results are shown in the tables below.

| | | Number of cfu per plate from dilution 10^{x} | | | | | | |
|---|---|---|---|---|---|---|---|---|
| | | Prevalue | | | Postvalue | | | |
| | Hand (L/R) | 10⁻³ | 10⁻⁴ | 10⁻⁵ | 10⁻⁰ | 10⁻¹ | 10⁻² | 10⁻³ |
| Test Formulation Sample 1 | L | >300 | >300 | 38 | 1874 | <15 | <15 | <15 |
| | R | >300 | >300 | 62 | 132 | 16 | <15 | <15 |
| Test Formulation Sample 2 | L | >300 | >300 | 101 | >300 | 63 | <15 | <15 |
| | R | >300 | >300 | 94 | >300 | 22 | <15 | <15 |
| Test Formulation Sample 3 | L | >300 | 46 | <15 | >300 | 93 | <15 | <15 |
| | R | >300 | 165 | <15 | >300 | 92 | <15 | <15 |
| Test Formulation Sample 4 | L | >300 | >300 | 48 | >300 | 69 | <15 | <15 |
| | R | >300 | >300 | 60 | >300 | 85 | <15 | <15 |
| Test Formulation Sample 5 | L | >300 | >300 | 54 | >300 | 287 | 27 | <15 |
| | R | >300 | >300 | 65 | >300 | 254 | 22 | <15 |
| Test Formulation Sample 6 | L | >300 | >300 | 66 | >300 | >300 | 119 | <15 |
| | R | >300 | >300 | 77 | >300 | >300 | 104 | <15 |
| Test Formulation Sample 7 | L | >300 | >300 | 50 | 68 | <15 | <15 | <15 |
| | R | >300 | >300 | 71 | 16 | <15 | <15 | <15 |
| Test Formulation Sample 8 | L | >300 | >300 | 85 | >300 | 89 | 16 | <15 |
| | R | >300 | >300 | 51 | >300 | 66 | <15 | <15 |
| EN1500 Reference Formulation Sample 1 | L | >300 | >300 | 46 | >300 | 91 | <15 | <15 |
| | R | >300 | >300 | 76 | >300 | 95 | <15 | <15 |
| EN1500 Reference Formulation Sample 2 | L | >300 | >300 | 91 | 164 | 17 | <15 | <15 |
| | R | >300 | >300 | 56 | 16 | <15 | <15 | <15 |
| EN1500 Reference Formulation Sample 3 | L | >300 | 16 | <15 | >300 | 131 | <15 | <15 |
| | R | >300 | 20 | <15 | >300 | 226 | 19 | <15 |
| EN1500 Reference Formulation Sample 4 | L | >300 | >300 | 39 | 154 | <15 | <15 | <15 |
| | R | >300 | >300 | 51 | >300 | 31 | <15 | <15 |
| EN1500 | L | 140 | 213 | 18 | >300 | 136 | <15 | <15 |
| Reference Formulation Sample 5 | | | | | | | | |
| | R | 144 | 151 | 16 | >300 | 69 | <15 | <15 |
| EN1500 Reference Formulation Sample 6 | L | >300 | >300 | 17 | >300 | >300 | 33 | <15 |
| | R | >300 | >300 | 158 | >300 | >300 | 38 | <15 |
| EN1500 Reference Formulation Sample 7 | L | >300 | 220 | 21 | 292 | 28 | <15 | <15 |
| | R | >300 | 210 | 28 | 36 | <15 | <15 | <15 |
| EN1500 Reference Formulation Sample 8 | L | >300 | >300 | 245 | 211 | 24 | <15 | <15 |
| | R | >300 | >300 | 98 | 28 | <15 | <15 | <15 |

| Sample No. | Comparative Formulation | | | Test Formulation | | |
|---|---|---|---|---|---|---|
| | Log prevalue | Log postvalue | Log Reduction Factor | Log prevalue | Log postvalue | Log Reduction Factor |
| 1 | 6.79 | 2.97 | 3.82 | 6.70 | 2.20 | 4.50 |
| 2 | 6.87 | 1.97 | 4.90 | 6.99 | 2.63 | 4.36 |
| 3 | 5.30 | 3.25 | 2.05 | 6.03 | 2.97 | 3.06 |
| 4 | 6.65 | 2.23 | 4.42 | 6.73 | 2.89 | 3.84 |
| 5 | 6.26 | 3.01 | 3.25 | 6.78 | 3.43 | 3.35 |
| 6 | 6.94 | 3.56 | 3.38 | 6.86 | 4.05 | 2.81 |
| 7 | 6.34 | 2.23 | 4.11 | 6.79 | 1.62 | 5.17 |
| 8 | 7.24 | 2.10 | 5.14 | 6.83 | 2.91 | 3.92 |
| Mean | 6.55 | 2.66 | 3.88 | 6.71 | 2.84 | 3.88 |
| Standard Deviation | 0.60 | 0.60 | 1.00 | 0.29 | 0.73 | 0.79 |

As shown in the tables, the test foaming formulation including the silicone-based polyurethane as the foaming agent was capable of disinfecting the hands of the test subjects as well as the EN1500 Reference foaming formulation.

### Example 4

In this Example, various copolymers for use in alcohol-based cleansing formulations were evaluated for their ability to foam.

Specifically, formulations including 65% by weight alcohol were prepared by mixing water, alcohol, and one of the copolymers shown in the table below. The formulations were then observed for formation of forming. The results are shown below.

| Trade Name | INCI Name | Wt% | Successful Foam Production (Y/N) |
|---|---|---|---|
| Microcare Silicone C1024 (Thor Personal Care, France) | Bis-Methoxy PEG-10 Dimethyl MEA/HDI/Bis-PEG-10 Dimethicone Copolymer | 0.5 | Y |
| Microcare Silicone C1024 (Thor Personal Care) | Bis-Methoxy PEG-10 Dimethyl MEA/HDI/Bis-PEG-10 Dimethicone Copolymer | 1.0 | Y |
| Microcare Silicone C1024 (Thor Personal Care) | Bis-Methoxy PEG-10 Dimethyl MEA/HDI/Bis-PEG-10 Dimethicone Copolymer | 3.0 | Y |
| Polyderm PPI-SI-WS (Alzo International Corporation, Sayreville, New Jersey) | Bis-PEG-15 Dimethicone/IPDI Copolymer | 1.0 | Y |
| Polyderm PPI-SI-WI (Alzo International Corporation) | Bis-PPG-15 Dimethicone/IPDI Copolymer | 1.0 | Y |
| Polyderm PPI-GH (Alzo International Corporation) | Glycereth-7 Hydroxystearate/IPDI Copolymer | 1.0 | N |
| Polyderm PPI-CA-15 (Alzo International Corporation) | Di-PEG-15 Cocamine/IPDI Copolymer | 1.0 | N |
| Polyderm PPI-SA-15 (Alzo International Corporation) | PEG-15 Soyamine/IPDI Copolymer | 1.0 | N |
| Polyderm PPI-G7/CA (Alzo International Corporation) | Glycereth-7-Diglycerol-PEG-15 Cocamine/IPDI Copolymer | 1.0 | N |
| Polyderm PPI-CO-40 (Alzo International Corporation) | PEG-40 Hydrogenated Castor Oil/IPDI Copolymer | 1.0 | N |
| Polynecon PPI-SA-15D (Alzo International Corporation) | PEG-15 Soyamine/IPDI Copolymer Dimer Dilinoleate | 1.0 | N |

As shown in the table, the only copolymers capable of providing foam to the alcohol-based cleansing formulations included Microcare Silicone C1024, Polyderm PPI-SI-WS, and Polyderm PPI-SI-WI, all of which include a polymeric backbone comprising at least one silaphilic moiety, at least one lipophilic moiety and at least one hydrophilic moiety.

When introducing elements of the present disclosure or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

In view of the above, it will be seen that the several objects of the disclosure are achieved and other advantageous results attained.

## Claims

1. A foaming formulation comprising water, an alcohol, and a silicone-based polyurethane having a polymeric backbone comprising at least one silaphilic moiety, at least one lipophilic moiety and at least one hydrophilic moiety; wherein the alcohol is present in the formulation in the amount of at least 50% by weight; wherein the polymeric backbone comprises a bis-(PEG)x dimethicone, wherein x ranges from 8 to 20, and the at least one lipophilic moiety includes a diisocyanate monomer.

2. The foaming formulation of claim 1, wherein the polymeric backbone is selected from the group consisting of isophorone diisocyanate and hexamethylene diisocyanate.

3. The foaming formulation of claim 1 comprising from 5.0% by weight to 45% by weight water, from 50% by weight to 90% by weight alcohol, and from 0.1% by weight to 10% by weight silicone-based polyurethane.

4. The foaming formulation of claim 1, wherein the alcohol is selected from the group consisting of methanol, ethanol, n-propanol, isopropanol, n-butanol, tert-butanol, 2-butanol, pentanol, hexanol, and combinations thereof.

5. The foaming formulation of claim 1 comprising from 60% by weight to 80% by weight alcohol.

6. The foaming formulation of claim 1 comprising from 0.5% by weight to 5.0% by weight silicone-based polyurethane, preferably from 1.0% by weight to 3.5% by weight silicone-based polyurethane.

7. The foaming formulation of claim 1 further comprising at least one agent selected from the group consisting of emollients, humectants, moisturizers, botanicals, foam stabilizers, vitamins, disinfectants, abrasives, non-aqueous solvents, preservatives, pH modifiers, sequestrants, antimicrobials, antioxidants, anti-reddening agents, astringents, deodorants, external analgesics, film formers, fragrances, hydrotropes, opacifiers, skin conditioning agents, skin exfoliating agents, skin protectants, sunscreens, and thickeners.

8. A process of preparing a foaming formulation, the process comprising mixing water, an alcohol, and a silicone-based polyurethane having a polymeric backbone comprising at least one silaphilic moiety, at least one lipophilic moiety and at least one hydrophilic moiety;
wherein the alcohol is present in the formulation in the amount of at least 50% by weight;
wherein the polymeric backbone comprises a bis-(PEG)x dimethicone, wherein x ranges from 8 to 20, and the at least one lipophilic moiety includes a diisocyanate monomer.

9. The process of claim 8, wherein from 5% by weight to 45% by weight of water, from 50% by weight to 90% by weight of the alcohol, and from 0.1% by weight to 10% by weight of the silicone-based polyurethane are mixed; preferably from 20% by weight to 30% by weight of water, from 65% by weight to 75% by weight of the alcohol, and from 1.0% by weight to 3.5% by weight of the silicone-based polyurethane are mixed.

10. A method for cleansing comprising applying to a substrate a foaming formulation comprising water, an alcohol, and a silicone-based polyurethane having a polymeric backbone comprising at least one silaphilic moiety, at least one lipophilic moiety and at least one hydrophilic moiety;
wherein the alcohol is present in the formulation in the amount of at least 50% by weight;
wherein the polymeric backbone comprises a bis-(PEG)x dimethicone, wherein x ranges from 8 to 20, and the at least one lipophilic moiety includes a diisocyanate monomer.

11. The method of claim 10, wherein the foaming formulation comprises from 5.0% by weight to 45% by weight water, from 50% by weight to 90% by weight alcohol, and from 0.1% by weight to 10% by weight silicone-based polyurethane.

12. The method of claim 10, wherein the alcohol is selected from the group consisting of methanol, ethanol, n-propanol, isopropanol, n-butanol, tert-butanol, 2-butanol, pentanol, hexanol, and combinations thereof.

13. The method of claim 10, wherein the foaming formulation comprises from 0.5% by weight to 5.0% by weight silicone-based polyurethane, preferably from 1.0% by weight to 3.5% by weight silicone-based polyurethane.

14. The method of claim 10, wherein the substrate is skin.

15. The method of claim 10, wherein the foaming formulation further comprises at least one agent selected from the group consisting of emollients, humectants, moisturizers, botanicals, foam stabilizers, vitamins, disinfectants, abrasives, non-aqueous solvents, preservatives, pH modifiers, sequestrants, antimicrobials, antioxidants, anti-reddening agents, astringents, deodorants, external analgesics, film formers, fragrances, hydrotropes, opacifiers, skin conditioning agents, skin exfoliating agents, skin protectants, sunscreens, and thickeners.

## Patentansprüche

1. Schäumende Formulierung, umfassend Wasser, einen Alkohol und ein Polyurethan auf Silikonbasis mit einem polymeren Rückgrat, der wenigstens einen silaphilen Rest, wenigstens einen lipophilen Rest und wenigstens einen hydrophilen Rest umfasst;
wobei der Alkohol in der Formulierung in einer Menge von wenigstens 50 Gew.-% vorhanden ist; wobei das polymere Rückgrat ein Bis-(PEG)x-dimethicon umfasst, wobei x von 8 bis 20 reicht und der wenigstens eine lipophile Rest ein Diisocyanatmonomer einschließt.

2. Schäumende Formulierung nach Anspruch 1, wobei das polymere Rückgrat ausgewählt ist aus der Gruppe bestehend aus Isophorondiisocyanat und Hexamethylendiisocyanat.

3. Schäumende Formulierung nach Anspruch 1, umfassend von 5,0 Gew.-% bis 45 Gew.-% Wasser, von 50 Gew.-% bis 90 Gew.-% Alkohol und von 0,1 Gew.-% bis 10 Gew.-% eines Polyurethans auf Silikonbasis.

4. Schäumende Formulierung nach Anspruch 1, wobei der Alkohol ausgewählt ist aus der Gruppe bestehend aus Methanol, Ethanol, n-Propanol, Isopropanol, n-Butanol, tert-Butanol, 2-Butanol, Pentanol, Hexanol und Kombinationen davon.

5. Schäumende Formulierung nach Anspruch 1, umfassend von 60 Gew.-% bis 80 Gew.-% Alkohol.

6. Schäumende Formulierung nach Anspruch 1, umfassend von 0,5 Gew.-% bis 5,0 Gew.-% eines Polyurethans auf Silikonbasis, vorzugsweise von 1,0 Gew.-% bis 3,5 Gew.-% eines Polyurethans auf Silikonbasis.

7. Schäumende Formulierung nach Anspruch 1, weiterhin umfassend wenigstens ein Mittel, das ausgewählt ist aus der Gruppe bestehend aus Weichmachern, Feuchthaltemitteln, Moisturizern, pflanzlichen Stoffen, Schaumstabilisatoren, Vitaminen, Desinfektionsmitteln, Scheuermitteln, nichtwässrigen Lösungsmitteln, Konservierungsmitteln, pH-Reglern, Komplexbildnern, antimikrobiellen Mitteln, Antioxidantien, rötungshemmenden Mitteln, Adstringentien, Deodorants, Analgetika zur äußerlichen Anwendung, Filmbildnern, Duftstoffen, hydrotrophen Verbindungen, Trübungsmitteln, Hautkonditionierungsmitteln, Hautabschälmitteln, Hautschutzmitteln, Sonnenschutzmitteln und Verdickungsmitteln.

8. Verfahren zur Herstellung einer schäumenden Formulierung, wobei das Verfahren das Vermischen von Wasser, eines Alkohols und eines Polyurethans auf Silikonbasis mit einem polymeren Rückgrat, das wenigstens einen silaphilen Rest, wenigstens einem lipophilen Rest und wenigstens einem hydrophilen Rest umfasst, umfasst;
wobei der Alkohol in der Formulierung in einer Menge von wenigstens 50 Gew.-% vorhanden ist;
wobei das polymere Rückgrat ein Bis-(PEG)x-dimethicon umfasst, wobei x von 8 bis 20 reicht und der wenigstens eine lipophile Rest ein Diisocyanatmonomer einschließt.

9. Verfahren nach Anspruch 8, wobei von 5 Gew.-% bis 45 Gew.-% Wasser, von 50 Gew.-% bis 90 Gew.-% des Alkohols und von 0,1 Gew.-% bis 10 Gew.-% des Polyurethans auf Silikonbasis vermischt werden; vorzugsweise von 20 Gew.-% bis 30 Gew.-% Wasser, von 65 Gew.-% bis 75 Gew.-% des Alkohols und von 1,0 Gew.-% bis 3,5 Gew.-% des Polyurethans auf Silikonbasis vermischt werden.

10. Verfahren zur Reinigung, umfassend das Auftragen einer schäumenden Formulierung, die Wasser, einen Alkohol und ein Polyurethan auf Silikonbasis mit einem polymeren Rückgrat, das wenigstens einen silaphilen Rest, wenigstens einem lipophilen Rest und wenigstens einem hydrophilen Rest umfasst, auf ein Substrat umfasst;
wobei der Alkohol in der Formulierung in einer Menge von wenigstens 50 Gew.-% vorhanden ist;
wobei das polymere Rückgrat ein Bis-(PEG)x-dimethicon umfasst, wobei x von 8 bis 20 reicht und der wenigstens eine lipophile Rest ein Diisocyanatmonomer einschließt.

11. Verfahren nach Anspruch 10, wobei die schäumende Formulierung von 5,0 Gew.-% bis 45 Gew.-% Wasser, von 50 Gew.-% bis 90 Gew.-% Alkohol und von 0,1 Gew.-% bis 10 Gew.-% eines Polyurethans auf Silikonbasis umfasst.

12. Verfahren nach Anspruch 10, wobei der Alkohol ausgewählt ist aus der Gruppe bestehend aus Methanol, Ethanol, n-Propanol, Isopropanol, n-Butanol, tert-Butanol, 2-Butanol, Pentanol, Hexanol und Kombinationen davon.

13. Verfahren nach Anspruch 10, wobei die schäumende Formulierung von 0,5 Gew.-% bis 5,0 Gew.-% eines Polyurethans auf Silikonbasis, vorzugsweise von 1,0 Gew.-% bis 3,5 Gew.-% eines Polyurethans auf Silikonbasis umfasst.

14. Verfahren nach Anspruch 10, wobei das Substrat Haut ist.

15. Verfahren nach Anspruch 10, wobei die schäumende Formulierung weiterhin wenigstens ein Mittel umfasst, das ausgewählt ist aus der Gruppe bestehend aus Weichmachern, Feuchthaltemitteln, Moisturizern, pflanzlichen Stoffen, Schaumstabilisatoren, Vitaminen, Desinfektionsmitteln, Scheuermitteln, nichtwässrigen Lösungsmitteln, Konservierungsmitteln, pH-Reglern, Komplexbildnern, antimikrobiellen Mitteln, Antioxidantien, rötungshemmenden Mitteln, Adstringentien, Deodorants, Analgetika zur äußerlichen Anwendung, Filmbildnern, Duftstoffen, hydrotrophen Verbindungen, Trübungsmitteln, Hautkonditionierungsmitteln, Hautabschälmitteln, Hautschutzmitteln, Sonnenschutzmitteln und Verdickungsmitteln.

## Revendications

1. Formulation moussante contenant de l'eau, un alcool et un polyuréthane à base de silicone ayant un squelette polymère comprenant au moins un fragment silaphilique, au moins un fragment lipophile et au moins un fragment hydrophile ;
l'alcool étant présent dans la formulation dans la quantité d'au moins 50 % en poids ; le squelette polymère comprenant une bis-(PEG)x diméthicone, x valant de 8 à 20, et l'au moins un fragment lipophile comprenant un monomère de diisocyanate.

2. Formulation moussante de la revendication 1, dans laquelle le squelette polymère est choisi dans le groupe constitué par le diisocyanate d'isophorone et le diisocyanate d'hexaméthylène.

3. Formulation moussante de la revendication 1, comprenant 5,0 % en poids à 45 % en poids d'eau, 50 % en poids à 90 % en poids d'alcool, et de 0,1 % en poids à 10 % en poids de polyuréthane à base de silicone.

4. Formulation moussante de la revendication 1, dans laquelle l'alcool est choisi parmi le groupe constitué par le méthanol, l'éthanol, le n-propanol, l'isopropanol, le n-butanol, le tert-butanol, le 2-butanol, le pentanol, l'hexanol et leurs combinaisons.

5. Formulation moussante de la revendication 1, comprenant 60 % en poids à 80 % en poids d'alcool.

6. Formulation moussante de la revendication 1, comprenant 0,5 % en poids à 5,0 % en poids de polyuréthane à base de silicone, de préférence 1,0 % en poids à 3,5 % en poids de polyuréthane à base de silicone.

7. Formulation moussante de la revendication 1, comprenant en outre au moins un agent choisi dans le groupe constitué par les émollients, les humectants, les hydratants, les produits végétaux, les stabilisants de mousse, les vitamines, les désinfectants, les abrasifs, les solvants non aqueux, les conservateurs, les modificateurs de pH, les séquestrants, les antimicrobiens, les antioxydants, les agents anti-rougeurs, les astringents, les déodorants, les analgésiques externes, les agents filmogènes, les parfums, les hydrotropes, les opacifiants, les agents de soin cutané, les agents exfoliants, les agents de protection cutanée, les écrans solaires et les épaississants.

8. Processus de préparation d'une formulation moussante, le processus comprenant le mélange d'eau, d'un alcool et d'un polyuréthane à base de silicone ayant un squelette polymère comprenant au moins un fragment silaphilique, au moins un fragment lipophile et au moins un fragment hydrophile ;
l'alcool étant présent dans la formulation dans la quantité d'au moins 50 % en poids ;
le squelette polymère comprenant une bis-(PEG)x diméthicone, x valant de 8 à 20, et l'au moins un fragment lipophile comprenant un monomère de diisocyanate.

9. Processus de la revendication 8, dans lequel sont mélangés 5 % en poids à 45 % en poids d'eau, 50 % en poids à 90 % en poids de l'alcool et 0,1 % en poids à 10 % en poids du polyuréthane à base de silicone ; de préférence sont mélangés 20 % en poids à 30 % en poids d'eau, 65 % en poids à 75 % en poids de l'alcool et 1,0 % en poids à 3,5 % en poids du polyuréthane à base de silicone.

10. Procédé de nettoyage comprenant l'application sur un substrat d'une formulation moussante comprenant de l'eau, un alcool et un polyuréthane à base de silicone ayant un squelette polymère comprenant au moins un fragment silaphilique, au moins un fragment lipophile et au moins un fragment hydrophile ;
l'alcool étant présent dans la formulation dans la quantité d'au moins 50 % en poids ;
le squelette polymère comprenant une bis-(PEG)x diméthicone, x valant 8 à 20, et l'au moins un fragment lipophile comprenant un monomère de diisocyanate.

11. Procédé de la revendication 10, dans lequel la formulation moussante comprend 5,0 % en poids à 45 % en poids d'eau, 50 % en poids à 90 % en poids d'alcool, et de 0,1 % en poids à 10 % en poids de polyuréthane à base de silicone.

12. Procédé de la revendication 10, dans lequel l'alcool est choisi dans le groupe constitué par le méthanol, l'éthanol, le n-propanol, l'isopropanol, le n-butanol, le tert-butanol, le 2-butanol, le pentanol, l'hexanol et leurs combinaisons.

13. Procédé de la revendication 10, dans lequel la formulation moussante comprend 0,5 % en poids à 5,0 % en poids de polyuréthane à base de silicone, de préférence 1,0 % en poids à 3,5 % en poids de polyuréthane à base de silicone.

14. Procédé de la revendication 10, dans lequel le substrat est la peau.

15. Procédé de la revendication 10, dans lequel la formulation moussante comprend en outre au moins un agent choisi dans le groupe constitué par les émollients, les humectants, les hydratants, les produits végétaux, les stabilisants de mousse, les vitamines, les désinfectants, les abrasifs, les solvants non aqueux, les conservateurs, les modificateurs de pH, les séquestrants, les antimicrobiens, les antioxydants, les agents anti-rougeurs, les astringents, les déodorants, les analgésiques externes, les agents filmogènes, les parfums, les hydrotropes, les opacifiants, les agents de soin cutané, les agents exfoliants, les agents de protection cutanée, les écrans solaires et les épaississants.
